(19) Europäisches Patentamt
European Patent Office
Office européen des brevets

(11) **EP 2 187 767 B1**

(12) **EUROPEAN PATENT SPECIFICATION**

(45) Date of publication and mention
of the grant of the patent:
**19.10.2011 Bulletin 2011/42**

(51) Int Cl.:
*A23L 1/30* (2006.01)    *A23L 1/305* (2006.01)
*A23L 1/05* (2006.01)    *A23L 1/052* (2006.01)
*A23L 1/308* (2006.01)    *A61K 31/198* (2006.01)
*A61K 47/36* (2006.01)

(21) Application number: **08785545.8**

(22) Date of filing: **14.08.2008**

(86) International application number:
**PCT/EP2008/006690**

(87) International publication number:
**WO 2009/021735 (19.02.2009 Gazette 2009/08)**

(54) **INCREASED CREATINE RETENTION WITH SOLUBLE FIBRES**

ERHÖHTE KREATINRETENTION BEI LÖSLICHEN FASERN

RÉTENTION DE CRÉATINE ACCRUE AVEC DES FIBRES SOLUBLES

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **15.08.2007 EP 07114358**

(43) Date of publication of application:
**26.05.2010 Bulletin 2010/21**

(73) Proprietor: **Nestec S.A.**
**1800 Vevey (CH)**

(72) Inventors:
• **GERMAN, John, Bruce**
**CH-1072 Forel/Lavaux (CH)**
• **DECOMBAZ, Jacques, Edouard**
**CH-1806 St-Legier (CH)**
• **WUERSCH, Pierre**
**CH-1814 La Tour-de-Peilz (CH)**
• **VANDEPUTTE, Greet, Evelyne**
**CH-3280 Murten (CH)**

(74) Representative: **Oldroyd, Richard Duncan et al**
**Elkington and Fife LLP**
**Prospect House**
**8 Pembroke Road**
**Sevenoaks, Kent TN13 1XR (GB)**

(56) References cited:
**EP-A- 1 362 518        WO-A-01/28360**
**WO-A-01/35953        WO-A-94/02127**
**WO-A-2004/073420    WO-A-2005/020709**
**WO-A-2006/034586    WO-A-2008/115563**
**US-A- 5 908 864        US-A1- 2002 151 593**

• **DATABASE CAPLUS [Online] CHEMICAL
ABSTRACTS SERVICE, COLUMBUS, OHIO, US;
XP002509375 retrieved from STN Database
accession no. 2006:644620 & CN 1 792 257 A
(BEIJING COMPETITOR WEICHUANG SPORTS
TECHNOLOGY DEVELOPMENT CO.) 28 June
2006 (2006-06-28)**

**Description**

Field of the Invention

The present invention generally relates to increasing the efficiency of usage of an ingested biologically active substance by a body. In particular, the present invention relates to ways to improve the efficiency of utilization of ingested creatine in a body, in particular to increase the storage of creatine in the body and to slow down the excretion of creatine from the body. The present invention also relates to a food product having a composition comprising creatine and soluble fibres as active ingredients.

Background of the Invention

Creatine has become one of the most popular dietary supplements in the world. Creatine is known to improve sport performance during repeated short-duration/high-intensity exercises, and is found to amplify the increase in lean body mass and strength induced by resistance training. Creatine has also therapeutic benefits: it accelerates skeletal muscle recovery after prolonged immobilization, and slows down muscle wasting in certain myopathies. Furthermore, more recent information indicates that creatine may improve cognitive performance.

A large fraction of the ingested creatine is excreted in urine: from 33 to 77% of the original dose ranging from 8g/d to 21g/d during 1 to 14 days. This might potentially induce a kidney overload. A few renal impairments have been reported (Koshy KM, et al., N Engl J Med 340: 814-815), although cohort studies have not revealed any deleterious effects on renal functions (Francaux M, et al., Int J Sports Physiol Perf 1: 309-321). Recently, increased excretion of methylamine and formaldehyde have been observed after ingestion of large amounts of creatine (21g/d) for 2 weeks (Poortmans JR, et al., Med Sci Sports Exerc 37: 1717-1720). Although the level of these two mutagenic agents remained within the normal range for a healthy population, the development of strategies using low doses of creatine is wanted for reducing the potential risks induced by supplementation with larger amounts.

Therefore, a need exists for administering or supplying creatine in a way that its concentration in the blood of the consumer remains constant over a prolonged period of time.

There is also a need for administration of creatine in a way that prevents a "peak", that is, a transiently too high concentration of creatine in blood, because concentrations exceeding the saturation level of the creatine transporter into muscle might lead to more creatine being lost in the urine.

Furthermore, there is a need for consumable products that sustain an adequate concentration of creatine in blood in the range of its effectiveness. In other words, the blood concentration needs not only be maintained but maintained at a useful concentration.

In addition to this, particularly there is a need for a way to administer creatine in way that creatine is used by the body as efficiently as possible while avoiding that large amounts of creatine are excreted from the body unused.

Of course, if a mechanism is found that is suitable to alleviate the problems as set forth above, this mechanism must have no negative impact on the consumer and, furthermore, it preferably shouldn't interact with creatine. It would be an advantage, if a mechanism addressing these problems was based on natural substances the tolerance of which is not an issue and has not to be proven.

Overcoming these problems of the prior art and providing the art with a way to increase the efficiency of utilization of ingested creatine by the body is an object of the present invention. The present invention aims to increase the storage of ingested creatine in the body, in particular relative to the ingested creatine.

In WO 2005/020709 (NESTEC S.A.) a food product comprising soluble fibre and at least one exogenous active substance chosen from caffeine and/ or creatine is disclosed. The use of the mixture avoids short term peaks of the agent in blood serum, provides an increased storage of the agent in the body and slows down the excretion of the agent from the body.

Object and summary of the Invention

Surprisingly it was found that by administering creatine together with a viscous soluble fibre, in particular with viscous soluble fibres of high molecular weight in particular amounts will achieve these objects of the present invention.

Consequently, the object of the present invention may be achieved by a use in accordance with claim 1.

Importantly and unexpectedly, the present invention has the advantage that the amount of creatine lost by excretion is minimized.

The present invention describes the use of a combination comprising creatine and a viscous soluble fibre for preparation of a composition to increase the storage of creatine in the body and slow down the excretion of creatine from the body, wherein the viscous soluble fibres are high molecular weight soluble fibres having an average molecular weight of at least $1.7 \times 10^6$ g/mol.

The present invention also describes a food product having a composition comprising the following ingredients: about 2 to 40 % by weight of viscous soluble fibres, about 2 to 40 % by weight of creatine, about 5 to 45 % by weight of protein, about 1 to 20 % by weight of fats and/or oils and about 40 to 80 % by weight of carbohydrates, wherein the viscous soluble fibres are high molecular weight soluble fibres having an average molecular weight of at least $1.7 \times 10^6$ g/mol.

The present invention describes further a nutritional serving comprising a combination of creatine and a viscous soluble fibre having a high molecular weight of at least $1.7 \times 10^6$ g/mol.

Another advantage of the present invention is that it prevents a high and short-term peak of creatine concentration in blood with the effects described above. Furthermore, the possible high peak entailing undesired side effects of creatine are avoided, as well as a reduction of a creatine peak, e.g., blood plasma allows a more efficient usage of the ingested creatine by the body. Still another advantage of the present invention is that it provides a sustained concentration of creatine in blood and that it provides a concentration of creatine in blood plasma lying in the optimal range of effectiveness of the substance.

Another advantage is that many soluble fibres are known as food-components for a long time and may therefore be used without testing its tolerance in humans or animals Furthermore, the soluble fibre has a beneficial effect on the consumer. For example, it may be a prebiotic. Hence, two very different effects are achieved by using soluble fibre in a food product.

Furthermore, soluble fibres do not have to be isolated in order to exert the desired effect as described above. Natural products that contain soluble fibre, for example vegetables and cereals, for example oat and barley and their bran, are easily available and may directly be used.

Without wishing to be bound by theory the inventors believe that the addition of soluble fiber might slow down the velocity of intestinal creatine absorption and in addition to this also favour its retention by the body.

It has been found that the viscous soluble fibres may be selected such that retention of creatine in the body is increased. In particular it has been found that viscous high molecular weight fibres are particular effective for this purpose.

The present invention seeks to address the above-described problems. The invention also aims at other objects and particularly the solution of other problems as will appear in the rest of the present description.

The present invention allows a reduction in the amount of creatine excretion from the body relative to the amount of creatine ingested.

The use of the present invention allows not only a reduction in the overall amount of creatine lost due to excretion but also allows to slow down the excretion of creatine from the body. In one embodiment the use of the present invention slows down absorption rate of creatine in the gastro-intestinal tract. Short-term peaks of creatine in blood plasma are avoided and/or the blood plasma concentration of creatine is kept maintained for a prolonged time.

In an embodiment of the present invention, the food product of the invention is suitable for athletes, sportsmen and/or physically active people, because creatine is known as an ATP-recharger. A more efficient creatine usage will consequently provide a more efficient ATP-recharge system. One particular application of the subject matter of the present invention is to increase the performance of an athlete in team sports or during repeated sprints.

In the context of the present invention, the term "soluble fibre" means that fibre is at least 50% soluble according to the method of L. Prosky et al., J. Assoc, Off. Anal. Chem. 71, 1017-1023 (1988). For example the soluble fibre of the present invention may be selected from the group consisting of inulin, pectin, beta-glucan, gum arabic, tragacanth mucilages, guar and locust bean gum, agar, carageenans, alginates, xanthan or mixtures thereof. According to the present invention, viscous soluble fibers are used. Viscous soluble fibre may be any suitable soluble fibre, which is able to increase the viscosity of the contents of the stomach and the small intestine. Examples are beta - glucan, gums, such as guar gum, xanthan gum, and gum arabic, pectin, and, or mixtures of these. Beta-glucan is particularly preferred. As those of skill in the art will understand, the term "soluble fibre" comprises processed soluble fibre. It should be understood that a "soluble fibre" is a water extractable fibre.

It has been found that by incorporating viscous high molecular weight soluble fibres the intestinal absorption of creatine is slowed and the peak of plasma value in comparison with aqueous solution is reduced.

Viscosity developed in the gastro-intestinal tract during digestion has been found to be important for the retention of creatine in the body.

The invention provides a selection of viscous soluble fibres giving an improved viscosity, and thus an increased retention.

For the present invention, the digestion in the gastro-intestinal of a human has been simulated and the viscosity that the product provides after simulated digestion has been investigated.

It has been found that it is desirable to have a high viscosity, but it is not apparent from commercially available products which one would provide the highest viscosity in the intestine. The intestine has a certain pH, and other characteristic parameters.

Further, the food product will during the digestion process be subjected to mechanical stress which will break the product into pieces. By simulating the digestion of a complete product it has been possible to define a product composition which would lead to increase retention of creatine in the body.

Merely having high concentrations of the fibres in the products or using certain types of fibres do not answer the problem since an acceptable texture is needed, the product may be too hard or too soft.

At the same time the product needs to be processable and edible. If the concentration is increased the product should still be process able without the product resulting being too hard.

In an embodiment, the present invention proposes a product comprising a combination of creatine and high molecular weight soluble viscous fibres which provide the right viscosity in the intestinal environment to allow increased whole body retention of creatine.

It has been found that the fibres have an average molecular weight of at least $1.7 \times 10^6$ g/mol, more preferably $1.7 - 2.0 \times 10^6$ g/mol. Further, it has been found that the fibres should preferably be present in an amount of at least 1.3 g per serving.

Cereal β-glucans are linear homopolysaccharides of consecutively linked (1→4) β-D glucosyl residues that are separated by single (1→3) -linkages.

It has been shown that β-glucans improve glucose and insulin regulation and lower blood cholesterol. In addition, and for this study, the viscosity developed by β-glucan in the gastro-intestinal tract is of major importance.

A set of physico-chemical analysis has been set up to screen β-glucan concentrates and foods containing β-glucan to be able to select samples for clinical trials.

Firstly, the total β-glucan content is measured using a Megazyme assay kit for mixed linkage β-glucan.

Secondly, the amount and molecular weight of water-extractable (soluble) β-glucan is measured using a high-performance size-exlusion chromatography system (HPSEC) with detection based on the specific binding of calcofluor to β-glucan.

Thirdly, a method was developed to measure viscosity of β-glucan concentrates after simulated human gastric & duodenal digestion. β-glucan concentrate or food containing β-glucan concentrate is added to a filter bag containing simulated stomach juice (pH 2.5) without porcine pepsin solution. The material is mashed using a stomacher simulating the consumption of the food. Secondly, porcine pepsin solution is added. The material is incubated for 30 minutes at 37°C to simulate human gastric digestion. Afterwards, duodenal juice (pH 6.8) is added. The material is further incubated for 30 minutes at 37°C minutes duodenal digestion on a parallel plate variable shear viscometer.

Brief description of the drawings

Further features and advantages of the present invention are apparent from the following examples and figures.

Figure 1 summarizes the experimental protocol.

Figure 2 presents the percentage of initial dose of creatine (2g) excreted in urine in 24h. Creatine was ingested as an aqueous solution (AS), or incorporated in a beta-glucan- (BG) or protein- (PP) rich food bar. P-values above histograms depict statistical significances of differences with AS treatment.

Figure 3 shows the kinetics of plasma creatine after an ingestion of 2g creatine. The results are expressed as the means $\pm$ SEM (n=17). *P<0.05, #P<0.01, §P<0.001 vs AS treatment same time.

Figure 4 shows the kinetics of plasma creatine after an ingestion of 2g creatine. The fitted curve is given by equation 1 (see material and method section). AS, aqueous solution; BG, beta-glucan-rich bar; PP, protein-rich bar. The results are expressed as the means $\pm$ SEM (n=17).

Figure 5 presents the plasma creatine (a) and creatinine (b) concentrations measured before (black) and after one week (grey) creatine supplementation (3 x 2g per day). P-values above histograms depict statistical significances of differences between pre- and post- creatine supplementation. AS, aqueous solution; BG, beta-glucan-rich bar; PP, protein-rich bar. The results are expressed as the means $\pm$ SEM (n=17).

Figure 6 displays the erythrocyte creatine concentration measured before (black) and after one week (grey) creatine supplementation (3 x 2g per day). P-values above histograms depict statistical significances of differences between pre- and post- creatine supplementation. AS, aqueous solution; BG, beta-glucan-rich bar; PP, protein-rich bar. The results are expressed as the means $\pm$ SEM (n=17).

Figure 7 shows a diagram illustrating the viscosity after simulated human digestion with low and high molecular weight β-glucan concentrates.

Detailed description of embodiments

Although the present invention has been described with reference to preferred embodiments thereof, many modifications and alternations may be made by a person having ordinary skill in the art without departing from the scope of this invention which is defined by the appended claims.

The present invention relates to the use of a combination comprising creatine and a viscous soluble fibre for the preparation of a composition to increase the storage of creatine in the body and to slow down the excretion of creatine from the body, wherein the viscous soluble fibres are high molecular weight soluble fibres having an average molecular weight of at least 1.7 x $10^6$ g/mol.

The present invention also relates to the use of a combination comprising creatine and a viscous soluble fibre for the preparation of a composition to increase the efficiency of utilization of ingested creatine and to increase the ratio of creatine stored in the body to ingested creatine, wherein the viscous soluble fibres are high molecular weight soluble fibres having an average molecular weight of at least 1.7 x $10^6$ g/mol.

In particular during the first 24 hours after creatine uptake the creatine loss due to excretion may be minimized. In one embodiment it is possible to minimize the creatine loss by excretion to only 5-10 %. Consequently, e.g., 24 hours after creatine ingestion a ratio of creatine stored in the body to ingested creatine of about 90% to 95% may be achieved with the use of the present invention.

One embodiment of the use of the present invention is a therapeutic use.

In another embodiment the use of the present invention is a non-therapeutic use.

With the use of the present invention creatine is stored in the body preferably in the red blood cells and/or in muscle tissue. This ensures a high bioavailability of the ingested creatine and allows a very efficient usage wherever creatine is needed in the body. The present invention allows a reduction in the amount of creatine excreted from the body relative to the amount of creatine ingested.

The use of the present invention allows not only a reduction in the overall amount of creatine lost due to excretion but also allows to slow down the excretion of creatine from the body. In one embodiment the use of the present invention slows down absorption rate of creatine in the gastro-intestinal tract. Short-term peaks of creatine in blood plasma are avoided and/or the blood plasma concentration of creatine is maintained for a prolonged time.

The viscous soluble fibres are high molecular weight soluble fibres. Specifically, the high molecular weight soluble fibres have an average molecular weight of at least 1.7 x $10^6$ g/mol. More preferably, the viscous soluble fibres have an average molecular weight of 1.7 to 2.0 x $10^6$ g/mol.

In an embodiment of the present invention, the food product is suitable for athletes, sportsmen and/or physically active people, because creatine is known as an ATP-recharger. A more efficient creatine usage will consequently provide a more efficient ATP-recharge system. One particular application of the subject matter of the present invention is to increase the performance of an athlete in team sports or during repeated sprints.

The compositions prepared by the use of the present invention may be administered orally

While the composition for oral consumption may be a medicament or a food-product it is preferred that the composition is a food product. The food product may be selected from the group consisting of bars, in particular cereal bars, cereals, in particular breakfast cereals, dried powders for reconstitution with liquids, drinks comprising fiber, or sport gels.

It is possible to produce a food product, which may be liquid or solid, comprising the combination of soluble fibre and creatine as a solid or soft bar. The composition for oral consumption may also be in the form of a cookie, a biscuit, a snack, a cracker, a bread or a bread like product. It is likewise possible to add creatine and soluble fibre to other food products, such as dairy products, ice cream, confectionery or beverages.

For example, sufficient amounts of a powder of creatine may be added to whole grain oat and/or barley flour, enriched with de-fatted oat and/or barley bran to guarantee sufficient amount of viscous soluble fibre. This mixture may be used to form a bread dough.

If the product produced by the use of the present invention is intended to be a bar, there are a variety of possibilities to incorporate soluble fibre and creatine into a bar. In US 4,871,557 a method for obtaining a bar with supplemental dietary fibre is disclosed. The bar according to this reference is produced by extruding a fibre mixture containing apple fibre, corn bran, water and rice flour (the latter having the function of a binder), which is afterwards particle sized, i.e. shredded or ground to obtain flakes. These flakes high in fibre are then added to the other conventional ingredients of the bar. More precisely, the fibre mixture is added to other dry ingredients as grains (e.g. toasted rolled oats or crisped rice), nuts, fruits and/or chocolate chips. These dry ingredients are mixed with the extruded-shredded fibre-flakes and a (liquid) syrup blend is added. Other ingredients may be added and the mixture is transferred to a conventional bar forming line, which subjects the mixture to a forming, heating and a cutting procedure in order to obtain a bar.

This method may easily be modified in order to obtain the composition for oral consumption according to the present invention. For example, beta-glucan may be added in the form of oat and/or barley bran concentrate to the cereals prior to extrusion. Creatine may be added at the same time or later. For example creatine may be added to the syrup. A bar may also be prepared by mixing dry ingredients, which may be, besides soluble fibre, cereals, nuts, fruits, chocolate, berries, milk solids, for example. The dry ingredients of a bar may be later mixed with a binder, which is normally a syrup. The dry mixture will of a bar will usually comprise 40 to 90%, preferably 60 to 80% by weight of the total recipe, whereas the binder will account for the rest of it (60 to 30%, preferably 50 to 40% by weight).

Cereals may be just flour that is added to the dry mixture of the bar. However, cereals may be used in the form of crisps, flakes, puffs (e.g., oven or gun puffed), extruded and/or extruded-expanded cereals may serve as an example. Cereals are wheat, maize, barley, oat, rice, oat, millet and the like. Hence, cereals comprise, for example, rice or maize crisps, puffed rice or oat, any kind of flakes, baked or compressed and flaked cereals and so forth. Depending on the density of the final bar, the cereals may be chosen accordingly. For example, if a light bar is preferred, it is better to use crisps and/or puffs as cereals, whereas when a dense bar is preferred, it may be better to use flakes or baked and compressed cereals, or simply flour, such as rice flour, for example.

If high amounts of oat and/or barley bran and/or oat and/or barley bran concentrate (in order to provide beta-glucan) are used to prepare a dry mixture for a bar, other cereals may be completely absent.

The dry mixture may also comprise nuts and fruits, for example. Examples are hazelnuts, wall-nuts, pecan-nuts, cashew nuts, almonds, coconut, chest nut, macadamia nut or mixtures of these. Nuts may be present in amounts up to 15%, preferably up to 10% by weight of the dry mixture. Fruits, such as apple, peach, pear, apricot, banana, orange, pine-apple, for example, and/or berries, such as raspberry, strawberry, blackberry, blueberry and the like may also be added to the dry mixture.

The binder to be added to the dry mixture may be syrup. A glucose syrup, for example, comprising a mixture of glucose and/or its polymers obtained by partial hydrolysis of starch, having a DE (dextrose equivalence) value of about 30 to 50 and a water content of from about 15 to 25% by weight of the binder. Generally, the binder may comprise invert sugar, glucose sugar and/or high fructose corn syrup, for example.

The binder may comprise milk solids, which are added in the form of milk powder and/or fresh milk. In the latter case, the addition of water may be at least partially replaced by the addition of milk. WO 0056171 describes a binder in this sense, which comprises 10 to 70 parts of sugar, 0.5 to 5 parts of a binding agent (a polysaccharide such as gum), up to 15 parts of glycerin, up to 60 parts of fruit pulp or concentrate, up to 10 parts of cocoa powder and added water up to a water content of from 10 to 30%, for example.

The binder may comprise 0 to 15%, preferably 0.5 to 5% glycerol. Glycerol is sometimes added to bars to provide a moist mouth-feel, while water content has generally to remain low to grant for a prolonged shelf life.

In a basic approach, the binder is simply a syrup comprising water (10 to 20% by weight of binder) and sugars, optionally further comprising fat (0 to 15%, preferably 2 to 10%), lecithin (0 to 1%, preferably 0.01 to 0.2%) and/or flavours, which is obtained by heating the water to 70 to 90 °C and adding/dissolving the sugars under stirring. Syrups with a water content of about 15 to 20% by weight are also commercially available.

Creatine may be added to the binder, or, alternatively, it may be added together with soluble fibre to the dry mixture, for example.

By mixing the dry mixture and the binder in the amounts mentioned above, a basic mass of the bar is obtained. This step may be done in any adequate mixing apparatus such as a screw mixer of the helical spring type with an axial sprinkling nozzle or with a coating drum, for example.

The basic mass may be transferred into a suitable forming or pressing apparatus, to shape the bar. For example, a Bepex-Hutt Roller Press type DP may be used, which pushes the basic mass through a slab nozzle or a strand nozzle, under pressures of up to 12 bar, preferably 5 to 10 bar. Another suitable apparatus is the Bepex-Hutt Roller Slab Former type GP, which does not imply such high pressures and therefore yields a flat paste with a lower specific weight.

However, the final bars may be obtained by other means, too. Generally, after forming the basic mass from the binder and the dry mixture, the further process may include forming rolling, pressure rolling, transfer on a pressure band, precooling (10 to 20 °C), cutting of the final shape, for example, by a slitter or guillotine cutter (from Sollich or Rademaker), second cooling (4 to 15 DEG C), and final packaging of the bar.

For example, if the cooked cereal product according to WO 9631128 is appropriately enriched with creatine, this may be a sufficient method to obtain the food product according to the present invention.

The food product as described above has a composition comprising, in percent by weight, 2 to 40% of viscous soluble fibre. Preferably, it comprises 3 to 30%, more preferably 5 to 20% of viscous soluble fibre. For example, the food product may comprise 7 to 16% viscous soluble fibre. Viscous soluble fibre may be derived from any suitable source as mentioned above.

Beta-glucan may be derived from any source known to contain beta-glucan including oat bran concentrate or a barley flour; more preferably a de-fatted oat bran or barley flour.

In this specification, a "de-fatted oat and/or barley bran concentrate" means an oat and/or barley bran fraction which has a beta-glucan content of above 10% by weight and which has been subjected to a process to remove, at least partially, oil and fats from the fraction. Ordinarily, oat and/or barley bran concentrates have a fat or oil content of greater than about 5% by weight. De-fatted oat and/or barley bran concentrates have an oil content of less than about 7% by weight; more usually about 4% to about 6% by weight. De-fatted oat and/or barley bran concentrates offer the advantage of better stability of the food product, easier processing, and improved texture and organoleptic properties of the food product.

De-fatted oat and/or barley bran concentrates of this type are commercially available; for example suitable oat bran concentrates may be purchased from Swedish Protein AB, Vrbacka, Sweden. Barley bran concentrates are e.g. obtainable from DKSH, Asia. Alternatively the oat and/or barley bran concentrate may be prepared by grinding dry oat and/or barley grains and then carefully screening the fibre material from the starchy components of the oat and/or barley grains. The fibre rich material may then be subjected to solvent extraction techniques to remove oils and fats from the material. A suitable procedure for the extraction of oils and fats is disclosed in British patent 1,526,553; the disclosure of which is incorporated by reference. The solvent extraction step may also be carried out prior to screening if desired. This screening and extraction procedure would be suitable for producing oat and/or barley bran concentrates with fibre contents at the lower end of the range; for example an oat and/or barley bran concentrate having a maximum beta -glucan content of about 15% by weight.

In a preferred embodiment, the food product according to the present invention comprises, in percent by weight of the raw ingredients, 20 to 80% oat and/or barley bran concentrate. Preferably, it comprises 30 to 70%, more preferably 40 to 60% oat and/or barley bran concentrate.

The food product according to the present invention further comprises, in percent by weight, 5 to 45% of protein, 1 to 20% of fats and/or oils and 40 to 80% of carbohydrates. In still a further embodiment, the food product according to the present invention comprises, in percent by weight, 5 to 20% of protein, 1 to 10% of fats and/or oils and 40 to 70% of carbohydrates. Preferably, the product comprises 10 to 15% of protein, 2 to 7% of fats and/or oils, and 50 to 60% of carbohydrates.

The protein, fats and carbohydrates may be of any plant or animal origin. For example, they may be comprised in cereals or other components used to manufacture the food product (see below), for example from oat and/or barley bran concentrate.

For optimal performance the composition prepared by the use of the present invention comprises preferably in percent by weight, about 2 to 40% of creatine, more preferably 10-35 % of creatine, even more preferred 20-30 % of creatine.

The compositions prepared by the use of the present invention are preferably ingested as follows: about 20g of creatine are ingested every day for 5 days. This will increase the creatine content in muscle by about 20%. Thereafter, about 3-5g are consumed daily to maintain the uptake of creatine by the muscles. The initial loading phase may also be omitted. In this case, the rise in muscle creatine content is slower, but reaches the same level after about 15 days. Other cell types, such as erythrocytes and neurons, also store creatine during supplementation.

Accordingly, the composition prepared by the use of the present invention may be consumed in a way that about 1.5-7g, preferably about 3-5g creatine per 75kg body weight are consumed per day. Optionally, the composition may be consumed in a way that about 15-25g, preferably about 18-22g of creatine per 75kg body weight are consumed per day during the first 1-7 days.

The composition prepared by the use of the present invention may have an energy content of about 75-200 kcal, preferably or about 100-180 kcal. Preferably, the composition may be consumed in the framework of a total diet that contains about 1500-3000 kcal per day, while even more preferably 1800-2500 kcal are consumed per day. Hence, in one embodiment the energy of the composition prepared by the use of the present invention should represent about 2 - 20 %, preferably 5-15 %, even more preferred 8-12 % of the total energy consumed during a day.

The food product of the present invention comprises the viscous soluble fibres in an amount of about 2 to 40 % by weight. The viscous soluble fibres are high molecular weight soluble fibres. The high molecular weight soluble fibres preferably have an average molecular weight of at least $1.7 \times 10^6$ g/mol, more preferred, in a range of $1.7 - 2.0 \times 10^6$ g/mol.

A nutritional serving according to the present invention comprises a combination of creatine and a viscous soluble fibre having a high molecular weight of at least $1.7 \times 10^6$ g/mol, preferably $1.7 - 2.0 \times 10^6$ g/mol, and, wherein a serving comprises at least 1.3 g of viscous high molecular weight soluble fibres and the soluble fibres provide a viscosity of a least 3000 mPa in a simulated gastro-intestinal digestion.

It is clear to those skilled in the art that they can combine freely all features disclosed herein without departing from the scope of the invention as originally disclosed herein.

The following examples illustrate the present invention.

Examples

Example 1

*Subjects*

Seventeen healthy males (21 $\pm$ 0.3 years, 179 $\pm$ 1.0cm, 71 $\pm$ 2.9kg, mean $\pm$ SEM) were recruited. They were physically active, although none of them was a high level athlete. They were instructed to keep their diet and their physical activity unchanged during the experiment. Prior to the beginning of the experiment, food intake was recorded during 7 days by means of a standardized questionnaire and computed using the Nutrilog software (Marans, France). Daily energy intake was 2447 $\pm$ 111.7kcal divided into 48 $\pm$ 1.1% carbohydrates, 34 $\pm$ 1.1% lipids, 3 $\pm$ 0.8% alcohol and 15 $\pm$ 1.1% proteins which correspond to 1.3 $\pm$ 0.10g of proteins per kg body mass. The subjects did not consume any food supplement for at least 2 months and they did not take any drug on a regular basis. They were given an oral and written account of the study before signing an informed consent document. All the procedures used were in accordance with the Declaration of Helsinki.

*Protocol*

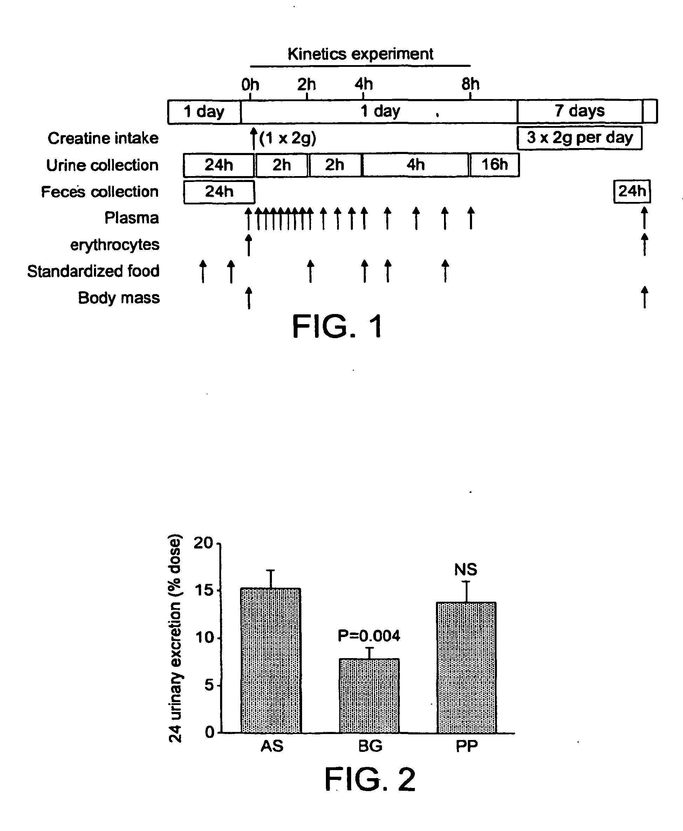

The subjects received randomly three treatments which were interspaced by a wash-out period of 40.0 $\pm$ 1.2 days, with a minimum of 29 days. One treatment consisted in 2g creatine powder (2.3 g creatine monohydrate, food grade, Creapure™), consumed in 150 ml of aqueous solution (AS). The two others were food bars with commercial recipes manufactured for the study by the Nestlé Product Technology Center PTC-Orbe, Switzerland: one was a protein-rich bar (PP) and the other one was a beta-glucan-rich bar (BG). The protocol is summarized in figure 1. The BG-bar contained 16.7g carbohydrates, 4.1g proteins, 2.3g lipids and 3.2g fibers (~1.5g beta-glucans) and a total energy content of 104kcal. The PP-bar contained 19.0g carbohydrates, 14.3g proteins, 1.3g lipids and 0.1g fibers and a total energy content of 144kcal. Both bars contained also 2g of creatine, as checked by subsequent analysis, incorporated as monohydrate at manufacture.

The day before the experiment, the subjects received standardized meals; they were asked to collect their 24h urine production. Following an overnight fast, they arrived at 7.00 a.m. in the laboratory where weighed. They filled in a binary (yes/no) questionnaire assessing the occurrence of adverse symptoms over the past 7 days (stomach ache, diarrhoea, constipation, nausea, headache, dizzy spell, black out, pounding, insomnia, tendonitis, cramps, muscle soreness, fatigue, loss of appetite, hunger, other complaints). A catheter was introduced into an antecubital vein and a first blood sample was drawn to determine the basal level of creatine and creatinine in plasma and erythrocyte. Then, the subjects ingested one of the three creatine products (AS, BG or PP) and 16 blood samples were further taken up during the next 8h to determine the plasma kinetics of creatine and creatinine. A standardized breakfast, snack, lunch and a last snack were given respectively 2h, 4h, 5h and 7h after creatine ingestion. Similarly, urines were collected 2h, 4h, 8h and 24h after creatine ingestion. The subjects were instructed to consume the test product 3 times a day, in the morning, at noon and in the evening, for the next 7 days. On the eighth day, the subjects returned to the laboratory after an overnight fast. They were weighed again and a blood sample was drawn for plasma and erythrocyte creatine and creatinine determination. The health status questionnaire was filled in again. Four of the subjects collected their 24h feces on six occasions: the day before and the last day of each supplementation period.

*Procedures*

Venous blood was collected on heparin as an anticoagulant. The tubes were placed immediately on ice and centrifuged at 3000g for 6min at 4°C. Plasma (900$\mu$L) was deproteinized with sulfosalicylic acid 5% (300$\mu$L) and centrifuged again at 3000g for 6min. NaOH 0.1M (90$\mu$L) was added to supernatant (300$\mu$L) to obtain a pH value of about 7.8. Samples were stored at -20°C.

After the first centrifugation, erythrocytes were washed twice with a similar volume of physiological water (0.9% NaCl, centrifugation: 1000g, 5min, 4°C). After three frozen and defrozen procedures, erythrocytes (300$\mu$L) were deproteinized with sulfosalicylic acid 5% (600$\mu$L) and then centrifuged at 3000g during 6min. Supernatant was collected and centrifuged again. The clear supernatant was neutralized with NaOH 0.1M.

Urines were collected in a plastic bottle and the volume measured. An aliquot (10mL) was stored at -20°C.

Creatine and creatinine content in plasma, erythrocytes and urine was analyzed by spectrophotometry by enzymatic techniques ( Siedel J et al., (1984) Clin Chem 30: 968), omitting creatininase for creatine determination. Creatine and creatinine content in test bars were determined by HPLC following homogenization and solvent extraction according to a modified method of Dash and Sawhney (Dash AK, Sawhney A (2002) J Pharm Biomed Anal 29: 939-945). Collection of 24h feces was placed in a plastic box and weighed. An aliquot was stored at -80°C. Due to the large bacterial content, the feces were prepared as soon as possible, avoiding the acidic deproteinization step that might induce a degradation of creatine to creatinine ( Ganguly S, et al., (2003) AAPS PharmSciTech 4: E25).

Both creatine and creatinine content in feces was analyzed by HPLC at 205nm. After dilution of a feces aliquot in 0.045M ammonium sulfate containing an internal standard (4-[2-aminoethyl]-benzene sulfonamide), the sample was filtered on a 10kDa membrane. The following conditions were used to identify creatine: 2 Beckman RP-C18 Ultrasphere ODS columns 5$\mu$ 25cm*4.6mm; elution: A/B (70/30) with 0.05% anhydrous trifluoroacetic acid (TFA) at 1.0mL/min; A: 0.1% $H_3PO_4$ in deionised $H_2O$; B: 1.4mg sodium dodecylsulfate/mL acetonitril. Spectral profiling (Diod Array Detector series 200 HP) demonstrated that the small peak corresponding to creatine in the samples was heterogeneous. The values reported for creatine in the results section correspond to highest possible estimates including a probable overestimation. Creatinine was detected according to another modified method ((Dash AK, Sawhney A (2002) J Pharm Biomed Anal 29: 939-945) using the same columns as for creatine but a different elution: ammonium sulfate 5.95g/L deionised $H_2O$ at 1.0mL/min. Due to the nature of the elution solvent and the small size of the creatinine peak, its spectral profile could not be obtained. As for creatine, the reported values correspond to highest possible estimates.

*Pharmacokinetics*

Using a Marquardt algorithm, all kinetics were fitted with the following equation:

```
C=    (FD/V*(ka/(ka-kel)) * (exp(-kel*(t-tlag))- exp(-
 ka*(t-tlag)))) (equation 1)
```

where C is the plasma creatine concentration minus the basal concentration ($\mu$M)
if C 0, then data were removed
F is the bioavailability
D is the dose (2g or 15 300 $\mu$mol)
V is the volume of distribution
ka is the velocity constant of absorption (first order, $h^{-1}$)
kel is the velocity constant of elimination (first order, $h^{-1}$)
t is the time (h)
tlag is the lag-time (h)

No weight was applied to data (weight = 1) and the initial parameters values were estimated using a Simplex algorithm.

This equation corresponds to a one-open compartmental model in which ka, kel and tlag are the primary parameters. Knowing their value, new kinetic parameters of plasma creatine were calculated:

$AUC_{0,\infty}$: the area under the curve integrated from zero to infinity

Since the bioavailability (F) may not be measured, only the apparent volume of distribution and the apparent clearance can be estimated:

Vd: the apparent volume of distribution (Vd = V/F, L)
Cl: the apparent clearance (Cl = Kel·Vd, L.$h^{-1}$)

*Statistics*

Results are presented as the means $\pm$ standard errors of the means (SEM). Differences between mean values of variables measured with the three forms of creatine ingestion (AS/BG/PP) were assessed by a mixed model representing the order of the treatments (first, second or third treatment) and when applicable, the time of measurement (pre- and post-treatment). When the variable tested was not affected by the order, either a one-way (single dose) or a two-way ANOVA (multiple doses) for repeated measures was used. When a one-way ANOVA was used, the Dunnett test was used as post-hoc test to compare BG and PP with AS as the reference treatment. In the case of a two-way ANOVA and the Bonferroni test was applied as post-hoc test to compare pre- and post-treatment conditions. Statistical significance of changes observed in frequencies of discrete variables was inferred by means of a Fisher-test. The significant threshold was set at $P < 0.05$.

Results

*Kinetics after a single dose*

*Urines*

In basal conditions, 24h urinary excretion of creatine was 2 ± 1.9mg. When a single dose of 2g creatine was consumed under the form of AS and PP, 15 ± 1.9% and 14 ± 2.2% of the dose, respectively, was found in urine collected during the first 24h after ingestion (Fig. 2). Less creatine was excreted after the BG treatment (8 ± 1.2%, P=0.004), as compared to AS and PP forms. Most of the excreted creatine appeared in urine during the first 2h following intake of AS (Table 1).

Table 1. Creatine urinary excretion

|  | 0-2h | 2-4h | 4-8h | 8-24h |
|---|---|---|---|---|
| AS | 0.193 ± 0.0204 | 0.107 ± 0.0217 | 0.002 ± 0.0010 | 0.001 ± 0.0001 |
| BG | 0.035 ± 0.0087*** | 0.108 ± 0.0195 | 0.010 ± 0.0051 | 0.001 ± 0.0003 |
| PP | 0.152 ± 0.0250 | 0.117 ± 0.0223 | 0.004 ± 0.0022 | 0.002 ± 0.0009 |

Urinary creatine excretion (g) during the first two hours, between the 2nd and the 4th hour, between the 4th and the 8th hour and between the 8th and the 24th hour after ingestion of 2g creatine under the form of an aqueous solution (AS), or incorporated in a beta-glucan- (BG) or protein- (PP) rich food bar. Results are expressed as the means ± SEM (n=17). ***P<0.001 vs AS treatment.

After 4h, creatine excretion values became similar to basal conditions. Creatinine urinary excretion was slightly less than 1g/24h and not affected by ingestion of 2g creatine whatever the form of ingestion. Neither creatine, nor creatinine excretions were affected by the order of the treatment.

*Plasma concentrations*

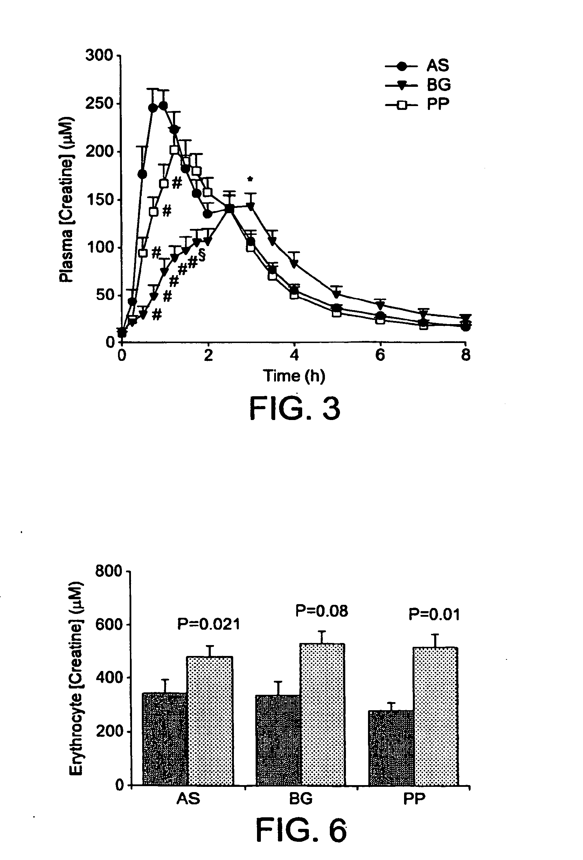

The mean basal plasma creatine concentration was 10 ± 1.0μM (Fig. 3). After ingestion of 2g creatine under the AS form, plasma concentration increased sharply to reach a peak (299 ± 19.5μM) in less than 1h (Table 2).

Table 2. Plasma kinetic data

|  | AS | BG | PP |
|---|---|---|---|
| Peak concentration of plasma creatine | 299 ± 19.5 | 174 ± 14.0*** | 237 ± 22.3* |
| (observed, μM) | 0.81 ± 0.050 | 2.40 ± 0.177*** | 1.31 0.146* |
| Time to peak concentration (observed, h) | 0.38 ± 0.033 | 0.78 ± 0.114*** | 0.43 ± 0.060 |
| Lag-time (h) | 23.63 ± 6.655 | 0.78 ± 0.045*** | 4.58 ± 7.050** |
| Velocity constant of absorption ($h^{-1}$) | 0.55 ± 0.150 | 4.67 ± 0.267*** | 2.18 ± 0.385*** |
| Time of absorption (h) | 0.68 ± 0.058 | 0.71 0.056 | 0.74 0.056 |
| Velocity constant of elimination ($h^{-1}$) | 587 ± 56.2 | ± 501 ± 44.9 | ± 528 ± 57.0 |
| Area under the curve ($0 \rightarrow \infty$ μM·h) | 47.1 ± 4.16 | 55.5 ± 8.88 | 60.6 ± 14.68 |
| Apparent volume of distribution (L) | 33.7 ± 6.22 | 35.4 ± 3.87 | 38.9 ± 6.70 |
| Apparent clearance ($L \cdot h^{-1}$) |  |  |  |

Plasma kinetic data after an ingestion of 2g creatine. AS, aqueous solution; BG, beta-glucan-rich bar; PP, protein-rich bar. The results are expressed as the means ± SEM (n=17). *P<0.05, ***P<0.001 vs AS treatment.

When the same amount of creatine was consumed as a bar, plasma concentration increase was slower; maximal plasma concentration was lower and delayed (Table 2). ANOVA design for repeated measures applied to the kinetic data showed a significant interaction between time and treatments (P<0.001) indicating different time-courses according to the ingested forms (AS/BG/PP). Post-hoc analyses showed that the difference emerged during the absorption phase before the peak concentrations were reached (Fig. 3). The peak creatine concentration was lower with BG (174 ± 14.0 μM) than with PP (237 ± 22.3 μM, P=0.019) and the peak concentration appeared later (2.40 ± 0.177h with BG vs 1.31 ± 0.15h with PP, P<0.001).

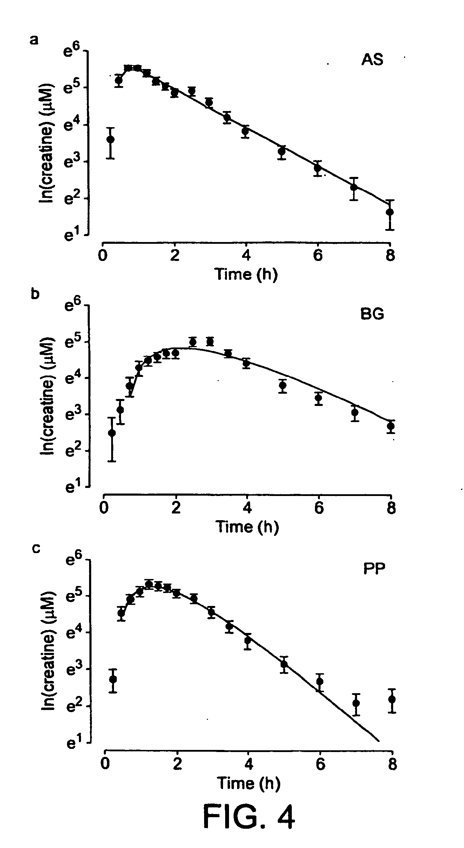

The one-open compartment model described by equation 1 yielded the best fitting of the plasma creatine time-course (Fig. 4). Other tested models did not allow the algorithm converging or gave larger residuals. Using equation 1 the median value of residuals was 1.06 $\mu$M in AS condition. The first and the third quartiles were -8.90 $\mu$M and 9.97 $\mu$M respectively. The distributions of residuals were not different in BG and PP conditions where the medians were -0.49 $\mu$M (25%: -10.04 $\mu$M - 75%: 8.67 $\mu$M) and 0.04 $\mu$M (25%: -10.13 $\mu$M - 75%: 7.98 $\mu$M), respectively. The relationships between observed and calculated plasma creatine concentrations were linear and explained a high fraction of variances: 96% in AS, 88% in BG and 95% in PP condition.

The lag-time (tlag), the velocity constant of elimination (kel) and the velocity constant of absorption (ka) are the three calculated parameters of equation 1. The mixed model analysis did not reveal any effect of the order by which the treatment were given to the subjects, as far as tlag, kel and ka mean values are concerned.

The lag-time (tlag) corresponds to the time necessary to observe a change in plasma creatine concentration after the bolus ingestion. When creatine was ingested under AS and PP forms, the tlag appeared 0.38 $\pm$ 0.033h and 0.43 $\pm$ 0.060h, respectively (P=NS), but tlag doubled when creatine was ingested as a BG bar (0.78 $\pm$ 0.114h, P<0.001, Table 2).

The velocity constant of absorption (ka) was faster when creatine was ingested under the form of AS in comparison with the bars (P<0.001, Table 2). The time of absorption (Tabs) is inversely related to ka. Tabs was 0.55 $\pm$ 0.150h in AS condition whereas it reached 2.18 $\pm$ 0.385h in PP (P<0.001) and 4.67 $\pm$ 0.267h in BG condition (P<0.001).

The mean half-life of elimination was 1.09 $\pm$ 0.06h. Neither the velocity constant of elimination, nor the apparent clearance were affected by the form of creatine ingested (Table 2). The apparent volume of distribution was about 75% of body mass and was not influenced by the form of creatine ingested (Table 2). The areas under the curve calculated by integration of equation 1 from 0 to infinity (AUC$_{0,\infty}$) were identical for all creatine forms (mean value: 539 $\pm$ 30.4 $\mu$M·h, Table 2).

Mean value of plasma creatinine concentrations in basal condition was 53.5 $\pm$ 1.50 $\mu$M. It changed by only a few $\mu$M during the eight hours following ingestion of creatine independently of the treatment.

*Ingestion of multiple doses*

*Plasma concentrations*

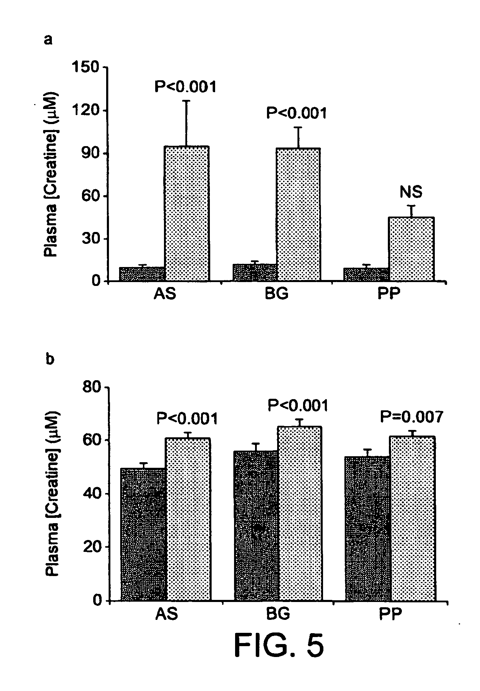

After one week creatine supplementation, plasma creatine and creatinine concentrations were measured again in a fasted state. Plasma creatine concentration, the value of which was not influenced by the order of the treatments, was 11 $\pm$ 2.5 fold higher after supplementation (Fig. 5A). The increase after PP creatine form was lower (+ 30 $\pm$ 5.2 $\mu$M) than after AS and BG supplementation (+ 86 $\pm$ 33.5 $\mu$M and + 85 $\pm$ 14.3 $\mu$M, respectively), with a significant interaction between treatments (AS/BG/PP) and conditions (pre-/post-supplementation). There was a significant order effect for creatinine plasma concentration (P<0.001). The basal mean values were 49.0 $\pm$ 2.22 $\mu$M, 54.7 $\pm$ 3.30 $\mu$M and 56.8 $\pm$ 1.83 kg for the 1st, 2nd and 3rd trial, respectively. Despite the progressive increase in the basal concentration over the time of the experimentation, one-week creatine supplementation increased slightly plasma creatinine concentrations by all treatments (P < 0.05), independently of the form of the ingested creatine form (Fig. 5B).

*Erythrocyte concentrations*

Erythrocyte creatine concentration showed large inter-individual differences as illustrated by a coefficient of variation of basal values of 59%, but was not influenced by the order of treatments.

Erythrocyte creatine concentration increased by 88 $\pm$ 22% after creatine supplementation independently of the form of ingested creatine (Fig. 6). There was no significant interaction between treatment (AS/BG/PP) and condition (pre-/post-) indicating that the form of creatine ingested had no further influence on the erythrocyte creatine level.

Basal erythrocyte creatinine concentration was about 25 $\mu$M and remained stable throughout the entire experiment.

*Feces*

Whatever the conditions, basal or supplemented, creatine and creatinine could not be quantified in human feces. The methods used were sufficiently sensitive to indicate that the maximal estimated value was -15 $\pm$ 1.2 $\mu$g per gram fresh feces for creatinine and ~50 $\pm$ 5.7 $\mu$g per gram fresh feces for creatine.

*Side effects*

During the initial visit and after each period of creatine supplementation, all subjects filled in a questionnaire listing potential side effects. No difference was observed in the frequency of declared health troubles except for muscle cramps which were less frequently reported after creatine supplementation (P<0.05).

Example 2

Viscosity after simulated human digestion

Low molecular weight β-glucan concentrates and high molecular weight β-glucan concentrates have been used in the simulated human digestion as described above.
The β-glucan concentrates used in this experiment are as follows:

- Sample A: Low molecular weight β-glucan concentrate having a molecular weight of 0.3 x $10^6$ g/mol;
- Sample B: Low molecular weight β-glucan concentrates having a molecular weight of 0.3 x $10^6$ g/mol;
- Sample C: High molecular weight β-glucan concentrates having a molecular weight of 2.0 x $10^6$ g/mol;
- Sample D: High molecular weight β-glucan concentrates having a molecular weight of 1.7 x $10^6$ g/mol and
- Sample E: High molecular weight β-glucan concentrate having a molecular weight of 1.7 x $10^6$ g/mol.

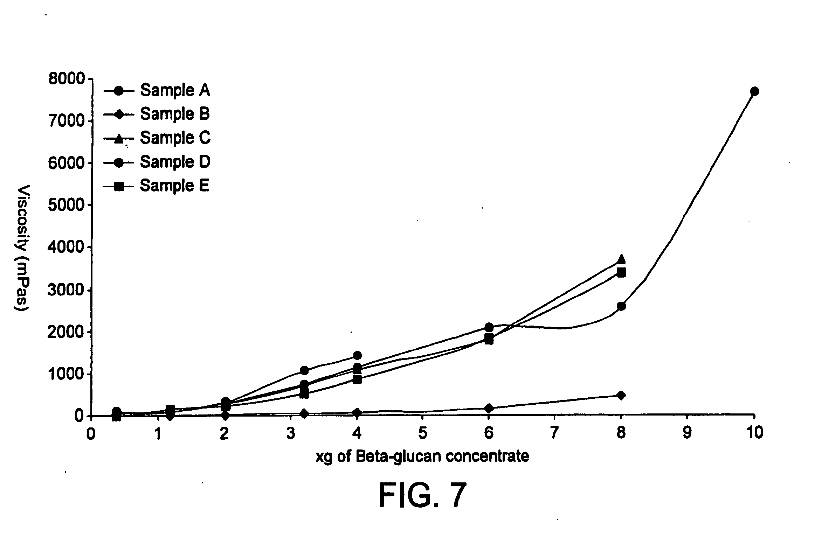

As can be taken from the diagram of Figure 7, sample A is not processable at high concentrations. Further, sample B does not develop a reasonable viscosity over a wide weight range. This means that sample B can only produce low viscosity bars. This concentrate is not preferred for producing products with a functionality of increasing the storage of creatine in the body and slow down the excretion of creatine from the body.
Samples C, D and E having a molecular weight of at least 1.7 x $10^6$ g/mol produce products having sufficient high viscosity to display the desired effects of creatine retention in the body.

**Claims**

1. Use of creatine and viscous soluble fibres for the preparation of a composition to increase the storage of creatine in the body and slow down the excretion of creatine from the body, wherein the viscous soluble fibres are high molecular weight soluble fibres having an average molecular weight of at least 1.7 x $10^6$ g/mol.

2. Use according to claim 1 wherein the viscous soluble fibres have an average molecular weight of 1.7-2.0 x $10^6$ g/mol.

3. Use according to any of claims 1-2, wherein creatine is stored in the red blood cells and in muscle tissue.

4. Use according to any of claims 1-3 to reduce the amount of creatine excretion from the body relative to the amount of creatine ingested.

5. Use according to any of claims 1-4 for slowing down absorption rate of creatine in the gastro-intestinal tract.

6. Use according to any of claims 1-5 to avoid short-term peaks of creatine in blood plasma and/or to keep the blood plasma concentration of creatine maintained for prolonged time.

7. Use according to any of claims 1-6, wherein the composition is suitable for pets or humans, in particular for athletes, sportsmen or physically active people.

8. Use according to any of claims 1-7, wherein the viscous soluble fiber is selected from the group consisting of inulin, pectin, beta-glucan, gum arabic, tragacanth mucilages, guar and locust bean gum, agar, carageenans, alginates, xanthan or mixtures thereof.

9. Use according to any of claims 1-8, wherein the composition is to be consumed in a way that 1.5-7g creatine per 75kg body weight are consumed per day.

10. Use according to any of claims 1-8, wherein the composition is to be consumed in a way that 15-25g of creatine per 75kg body weight are consumed per day during the first 1-7 days and subsequently 1.5-7g creatine per 75kg body weight are consumed per day.

11. Use according to any of claims 1-10, wherein the composition is to be consumed in a way that a total of 1500-3000 kcal are consumed per day.

**12.** Use according to any of claims 1-11, wherein the composition has an energy content of 75-300 kcal.

**13.** A food product having a composition comprising

- 2 to 40 % by weight of viscous soluble fibres;
- 2 to 40 % by weight of creatine,
- 5 to 45 % by weighty of protein,
- 1 to 20 % by weight of fats and/or oils and 40 to 80 % by weight of carbohydrates, and wherein the viscous soluble fibres are high molecular weight soluble fibres having an average molecular weight of at least $1.7 \times 10^6$ g/mol.

**14.** The food product according to claim 13 further comprising 20 to 80 % by weight of oat and/or barley bran concentrate.

**15.** The food product according to claims 13 or 14 wherein the viscous soluble fibres have an average molecular weight of $1.7\text{-}2.0 \times 10^6$ g/mol.

**16.** A nutritional serving comprising creatine and viscous soluble fibres having a molecular weight of at least $1.7 \times 10^6$ g/mol, preferably $1.7 - 2.0 \times 10^6$ g/mol, and, wherein a serving comprises at least 1.3 g of viscous high molecular weight soluble fibres and the soluble fibres provide a viscosity of at least 3000 mPa in a simulated gastro-intestinal digestion.

**Patentansprüche**

**1.** Verwendung von Kreatin und viskosen, löslichen Fasern bzw. Ballaststoff zur Herstellung einer Zusammensetzung, um die Speicherung von Kreatin im Körper zu erhöhen und die Ausscheidung von Kreatin aus dem Körper zu verzögern, wobei die viskosen, löslichen Fasern aus löslichen Fasern mit einem hohlen Molekulargewicht bestehen, welche ein durchschnittliches Molelculargewicht von mindestens $1{,}7 \times 10^6$ g/mol aufweisen.

**2.** Verwendung gemäß Anspruch 1, wobei die viskosen, löslichen Fasern ein durchschnittliches Molekulargewicht von $1{,}7\text{-}2{,}0 \times 10^6$ g/mol aufweisen.

**3.** Verwendung gemäß einem der Ansprüche 1 bis 2, wobei Kreatin in den roten Blutkörperchen und im Muskelgewebe gespeichert wird.

**4.** Verwendung gemäß einem der Ansprüche 1 bis 3, um die Menge an Kreatinausscheidung aus dem Körper relativ zu der Menge an aufgenommenem Kreatin zu reduzieren.

**5.** Verwendung gemäß einem der Ansprüche 1 bis 4 zur Verzögerung der Aufnahmegeschwindigkeit von Kreatin in den Magen-Darm-Trakt.

**6.** Verwendung gemäß einem der Ansprüche 1 bis 5, um kurzfristige Höchstwerte an Kreatin im Blutplasma zu vermeiden und/oder um die Konzentration von Kreatin im Blutplasma über längere Zeit aufrecht zu erhalten.

**7.** Verwendung gemäß einem der Ansprüche 1 bis 6, wobei die Zusammensetzung für Haustiere oder Menschen, insbesondere für Athleten, Sportler oder körperlich aktive Personen, geeignet ist.

**8.** Verwendung gemäß einem der Ansprüche 1 bis 7, wobei die viskose, lösliche Faser aus der Gruppe bestehend aus Inulin, Pektin, Beta-Glukan, Gummiarabicum, Tragantschleimstoffen, Guarkern- und Johannisbrotkernmehl, Agar, Carageenen, Alginaten, Xanthan oder Mischungen aus diesen ausgewählt ist.

**9.** Verwendung gemäß einem der Ansprüche 1 bis 8, wobei die Zusammensetzung auf eine Art und Weise konsumiert werden soll, dass täglich 1,5 bis 7g Kreatin pro 75 kg Körpergewicht konsumiert werden.

**10.** Verwendung gemäß einem der Ansprüche 1 bis 8, wobei die Zusammensetzung auf eine Art und Weise konsumiert werden soll, dass täglich 15 bis 25g Kreatin pro 75 kg Körpergewicht während der ersten 1 bis 7 Tage konsumiert werden und danach täglich 1,5 bis 7 g Kreatin pro 75 kg Körpergewicht konsumiert werden.

**11.** Verwendung gemäß einem der Ansprüche 1 bis 10, wobei die Zusammensetzung auf eine Art und Weise konsumiert werden soll, dass täglich insgesamt 1500 bis 3000 kcal konsumiert werden.

**12.** Verwendung gemäß einem der Ansprüche 1 bis 11, wobei die Zusammensetzung einen Energiegehalt von 75 bis 300 kcal aufweist.

**13.** Nahrungsmittelprodukt mit einer Zusammensetzung, welche

- 2 bis 40 Gewichtsprozent an viskosen, löslichen Fasern;
- 2 bis 40 Gewichtsprozent Kreatin,
- 5 bis 45 Gewichtsprozent Protein,
- 1 bis 20 Gewichtsprozent an Fetten und/oder Ölen sowie
40 bis 80 Gewichtsprozent an Kohlenhydraten aufweist, und wobei die viskosen, löslichen Fasern aus löslichen Fasern mit hohlem Molekulargewicht mit einem durchschnittlichen Molekulargewicht von mindestens $1{,}7 \times 10^6$ g/mol bestehen.

**14.** Nahrungsmittelprodukt gemäß Anspruch 13, welches weiterhin 20 bis 80 Gewichtsprozent Hafer- und/oder Gersienkleiekonzentrat aufweist.

**15.** Nahrungsmittelprodukt gemäß Anspruch 13 oder 14, wobei die viskosen, löslichen Fasern ein durchschnittliches Molekulargewicht von $1{,}7\text{-}2{,}0 \times 10^6$ g/mol aufweisen.

**16.** Ernährungsportion, welche Kreatin und viskose, lösliche Fasern mit einem Molekulargewicht von mindestens $1{,}7 \times 10^6$ g/mol, vorzugsweise $1{,}7\text{-}2{,}0 \times 10^6$ g/mol aufweist, und wobei eine Portion mindestens 1,3 g an viskosen, löslichen Fasern mit hohem Molekulargewicht aufweiset und die löslichen Fasern für eine Viskosität von mindestens 3000 mPa in einer simulierten Magen-Darm-Verdauung sorgen.

## Revendications

**1.** Utilisation de créatine et de fibres solubles visqueuses pour la préparation d'une composition pour augmenter le stockage de créatine dans le corps et ralentir l'excrétion de créatine du corps, dans laquelle les fibres solubles visqueuses sont des fibres solubles de poids moléculaire élevé ayant un poids moléculaire moyen d'au moins $1{,}7 \times 10^6$ g/mol.

**2.** Utilisation selon la revendication 1 dans laquelle les fibres solubles visqueuses ont un poids moléculaire moyen de $1{,}7\text{-}2{,}0 \times 10^6$ g/mol.

**3.** Utilisation selon l'une des revendications 1 ou 2, dans laquelle la créatine est stockée dans les cellules rouges du sang et dans le tissu musculaire.

**4.** Utilisation selon l'une des revendications 1 à 3 pour réduire la quantité de l'excrétion de créatine du corps par rapports à la quantité de créatine ingérée.

**5.** Utilisation selon l'une des revendications 1 à 4 pour ralentir le taux d'absorption de créatine dans le tractus gastro-intestinal.

**6.** Utilisation selon l'une des revendications 1 à 5 pour éviter des pics de courte durée de créatine dans le plasma sanguin et /ou pour garder la concentration de créatine dans le plasma sanguin maintenue pendant un temps prolongé.

**7.** Utilisation selon l'une des revendications 1 à 6, dans laquelle la composition est appropriée pour les animaux domestiques ou les humains, en particulier pour les athlètes, les sportifs ou les personnes physiquement actives.

**8.** Utilisation selon l'une des revendications 1 à 7, dans laquelle la fibre soluble visqueuse est sélectionnée parmi le groupe composé de l'inuline, la pectine, le beta-glucane, la gomme arabique, les mucilages adragantes, la gomme de guar et de caroube, l'agar-agar, les carraghénanes, les alginates, le xanthane ou les mélanges de ceux-ci.

9. Utilisation selon l'une des revendications 1 à 8, dans laquelle la composition est à consommer de manière à ce que 1,5-7 g de créatine pour 75 kg de poids corporel sont consommés par jour.

10. Utilisation selon l'une des revendications 1 à 8, dans laquelle la composition est à consommer de manière à ce que 15-25 g de créatine pour 75 kg de poids corporel sont consommés par jour pendant les premiers 1-7 jours et ensuite 1,5-7 g de créatine pour 75 kg de poids corporel sont consommées par jour.

11. Utilisation selon l'une des revendications 1 à 10, dans laquelle la composition est à consommer de manière à ce qu'un total de 1500-3000 kcal sont consommés par jour.

12. Utilisation selon l'une des revendications 1 à 11, dans laquelle la composition a un contenu énergétique de 75-300 kcal.

13. Un produit alimentaire ayant une composition comprenant :

   - de 2 à 40 % en poids de fibres solubles visqueuses ;
   - de 2 à 40 % en poids de créatine,
   - de 5 à 45 % en poids de protéine,
   - de 1 à 20 % en poids de matières grasses et /ou d'huiles et de 40 à 80 % en poids de glucides, et dans lequel les fibres solubles visqueuses sont des fibres solubles de poids moléculaire élevé ayant un poids moléculaire moyen d'au moins $1,7 \times 10^6$ g/mol.

14. Le produit alimentaire selon la revendication 13 comprenant en outre de 20 à 80 % en poids de concentré d'avoine et /ou de concentre de son d'orge.

15. Le produit alimentaire selon l'une des revendications 13 ou 14, dans lequel les fibres solubles visqueuses ont un poids moléculaire moyen de $1,7-2,0 \times 10^6$ g/mol.

16. Une portion nutritionnelle comprenant de la créatine et des fibres solubles visqueuses ayant un poids moléculaire d'au moins $1,7 \times 10^6$ g/mol, de préférence $1,7 - 2,0 \times 10^6$ g/mol et, dans laquelle une portion comprend au moins 1,3 g de fibres solubles visqueuses de poids moléculaire élevé et les fibres solubles fourbissent une viscosité d'au moins 3000 mPa dans une digestion gastro-intestinale simulée.

FIG. 1

FIG. 2

FIG. 3

FIG. 6

FIG. 4

FIG. 5

FIG. 7

EP 2 187 767 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005020709 A
- US 4871557 A
- WO 0056171 A

- WO 9631128 A
- GB 1526553 A


**Non-patent literature cited in the description**

- **Koshy KM et al.** *N Engl J Med,* vol. 340, 814-815
- **Francaux M et al.** *Int J Sports Physiol Perf,* vol. 1, 309-321
- **Poortmans JR et al.** *Med Sci Sports Exerc,* vol. 37, 1717-1720

- **L. Prosky et al.** *J. Assoc, Off. Anal. Chem.,* 1988, vol. 71, 1017-1023
- **Siedel J et al.** *Clin Chem,* 1984, vol. 30, 968
- **Dash AK ; Sawhney A.** *J Pharm Biomed Anal,* 2002, vol. 29, 939-945
- **Ganguly S et al.** *AAPS PharmSciTech,* 2003, vol. 4, E25